# EUROPEAN PATENT SPECIFICATION

(11) **EP 0 962 132 B1**
(45) Date of publication and mention of the grant of the patent: **14.05.2003**
(21) Application number: 99110590.9
(22) Date of filing: 01.06.1999
(51) Int. Cl.: A01M 1/20, A61L 9/03

(54) **Electric evaporator for insecticides or perfumes in liquid formulations with device to adjust the evaporation intensity**
Elektrischer Verdampfer für Insektizide oder Pärfume in Flüssigformulierung mit Vorrichtung zur Regelung der Dampfintensität
Evaporateur électrique pour insecticides et parfums sous forme liquide avec dispositif de réglage de l'intensité d'évaporation

(30) Priority: 05.06.1998 IT MI981276
(43) Date of publication of application: 08.12.1999
(73) Proprietor: ZOBELE HOLDING S.P.A., 38100 TRENTO (IT)
(72) Inventor: Zobele, Franco, 38100 Trento (IT)
(74) Representative: Faggioni, Marco, Dr. Ing.

(56) References cited:
- EP-A- 0 420 144
- WO-A-98/19526
- DE-U- 29 714 848
- GB-A- 2 194 442

## Description

The present invention concerns an electric evaporator for insecticides or perfumes in liquid formulations and, in particular, an evaporator of this type equipped with a device to obtain the adjustment of the evaporation intensity.

There are already known to be in this technical field the so-called adjustable evaporators, wherein the evaporation intensity of the active substance can be adjusted between a minimum and a maximum position, by changing the mutual positioning between the wick for the liquid formulation and the electric heating device. This type of evaporators is in fact meant to satisfy a specific market request to dispose of an evaporator apt to be easily adapted to special requirements of the user, introducing a number of considerable advantages for what concerns the working of the evaporator.

By adjusting the evaporation intensity, it is in fact possible to vary the amount of active principle introduced into the environment, according to the particular conditions thereof: for example, in the case of small rooms, or with a scarce change of air, the evaporation intensity can be kept lower than in the large or well ventilated rooms.

Moreover, the amount of active substance introduced into the environment can be varied according to the individual sensitiveness of each user, making the use of insecticides tolerable even into an environment with people who are particularly intolerant towards the same.

An adjustable evaporator of this type finally allows to perform an initial intense and thus rapid action to disinfest from insects or spread perfume into the environment, and then keep in the long run the effects of said action by introducing a reduced amount of active substance.

Consequently, the increased production costs of an evaporator with adjustable evaporation intensity, determined by its more complex structure, are widely repaid by its improved performances.

Various solutions have been proposed in technique up-to-date, to obtain an adjustment of the evaporation intensity. We shall not deal herein with the attempts made to adjust the heating intensity of the heating element with electric and/or electronic systems - since these are totally alien from the object of the present invention - but we shall merely deal with evaporators wherein the evaporation intensity is obtained by changing the mutual positioning between the heating device and the wick projecting from the bottle containing the liquid formulation. In particular, we shall deal only with evaporators wherein the bottle is the movable part. In fact the unacceptable drawback of the known evaporators wherein the movable part consists of the heating device, is to have to move, together with the heating device, also the respective connections and electric contacts; this, besides creating manufacturing difficulties, makes the whole mechanism delicate and more likely to undergo accidental breakages.

An evaporator of the aforecited type is for example that disclosed in the publication WO98/19526, in the name of the Applicant. In said evaporator, the adjustment of the evaporation intensity is obtained thanks to a partial rotation of the bottle containing the liquid formulation about its axis, in respect of a fixed circular bush; this solution is thus particularly successful when applied to bottles having a circular section.

Nevertheless, there has been a recent tendency on the market, substantially dictated by aesthetic reasons, towards the use of bottles with non-circular sections - for example, rectangular, trapezoidal or the like - which cannot however be utilized, in an equally efficient way, in an adjustable evaporator of the aforementioned type. If such bottles were in fact to be rotated about a central axis thereof, their lateral overall dimensions - due to the lack of symmetry of their section area - would continuously vary during rotation and it would thus be very difficult to find a shape, aesthetically acceptable, for the container meant to house such bottles.

According to a different technique - suited also for bottles with a non-circular section, but positively more rudimentary for what concerns its working - it has been provided to house the bottle by friction into a corresponding seat of the evaporator, the bottle being equipped with a lateral protuberance housed into a guide slot of the evaporator and the user acting on said protuberance to shift the bottle in respect of its seat.

However, in this second solution, the positioning of the bottle into its seat - and thus of the wick in respect of the heating device - is guaranteed merely by the friction existing between them. It can thus easily happen that the bottle undesirably slips out from its seat in the evaporator, or is anyhow shifted from the desired position.

Furthermore, to be able to check to what extent the bottle is inserted into the evaporator, one needs to see the position of its protuberance into the respective slot of the evaporator seat. This implies having access to the lateral lower portion of the evaporator, which turns out to be quite difficult when the same is plugged into a common current tap positioned just above the floor. Consequently, each time the evaporator requires an adjustment, the user must either bend down in an uncomfortable position, or else unplug the evaporator and thereby interrupt the working thereof.

Finally, since the sliding of the protuberance along the guide slot is obtained by finger pressure, it is neither pleasant to carry out nor easy to adjust. Moreover, only a very slight force can be applied to the protuberance, such as to make the adjustment difficult or even impossible when the friction between the bottle and the respective seat undesirably increases due to dirt or spilling of the liquid substance.

The object of the present invention is to thus supply an electric evaporator for liquid formulations - particularly liquid formulations contained in bottles having a non-circular section - wherein it may be possible to continuously change the position of the wick for the liquid formulation, in respect of the heating device of the evaporator, so as to obtain an adjustment of the evaporation intensity without having to cause any rotation of the bottle and, furthermore, with a comfortable and easily accessible control in any position of the evaporator.

Another object of the present invention is to supply an evaporator wherein the bottle is firmly and steadily held in position - in the desired position of adjustment - independently from any impacts or shakes which may be imparted on the evaporator.

According to the present invention, said objects are reached by means of an electric evaporator for insecticides or perfumes in liquid formulations - of the type comprising a housing, a rotable plug, an electric heating device and a bottle containing the liquid formulation, provided with a wick, wherein said bottle is held into a bottle holder, rectilinearly slidable in respect of the housing in a direction parallel to the axis of the wick, said bottle holder also comprising a cylindrical portion connected in a screw-and-nut relationship to a control bush apt to be operated externally to the evaporator so as to control the rectilinear sliding motion of said bottle holder.

The present invention will now be described in further detail, with reference to the accompanying drawings, in which:
Fig. 1 is a longitudinal section view of the evaporator according to the invention, without the bottle containing the liquid formulation;
Fig. 2 is a front elevation view of the evaporator shown in fig. 1;

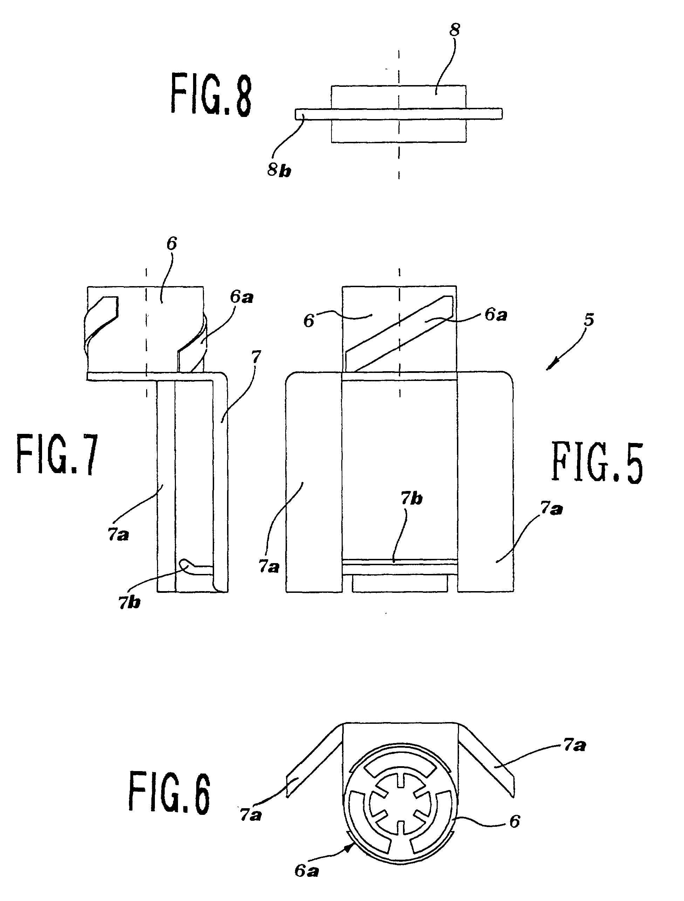

Fig. 3 is a rear elevation view of the evaporator shown in fig. 1;
Fig. 4 is a plan view of the evaporator shown in fig. 1;
Fig. 5 is a front view of the bottle holder;
Fig. 6 is a plan view of the bottle holder;
Fig. 7 is a lateral view of the bottle holder;
Fig. 8 is a front view of the control bush;
Fig. 9 is a cross section view of the bottle holder of fig. 7;
Fig. 10 is a cross section view of the control bush shown in fig. 8;
Fig. 11 is a cross section view of the elements shown in figs. 9 and 10, in a mounted position;
Figs. 12, 13 and 14 are views similar to that of fig. 1, showing three successive steps of the insertion of the bottle containing the liquid formulation into the evaporator; and
Figs. 15 and 16 are views similar to that of fig. 1, showing the bottle inserted in a position of maximum and, respectively, minimum evaporation intensity.

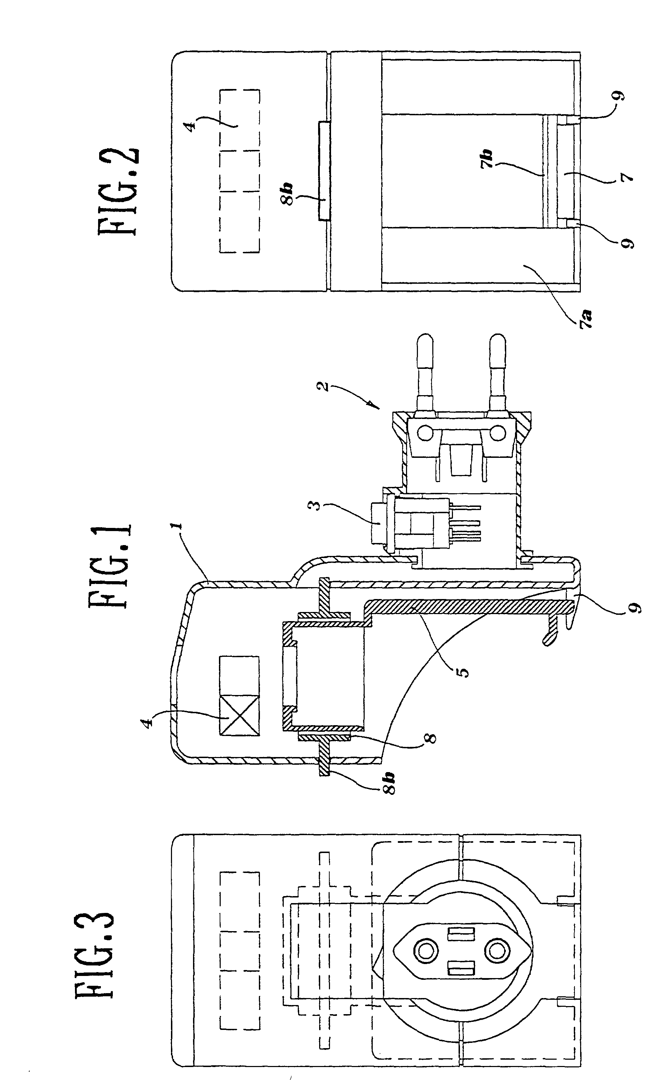

With reference to figs. 1 and 2, the evaporator according to the present invention consists of a housing 1 to which there is fixed, freely rotatable and in known manner, a unit comprising a plug 2 and a switch 3, suited to allow plugging the evaporator both into horizontal and into vertical current taps.

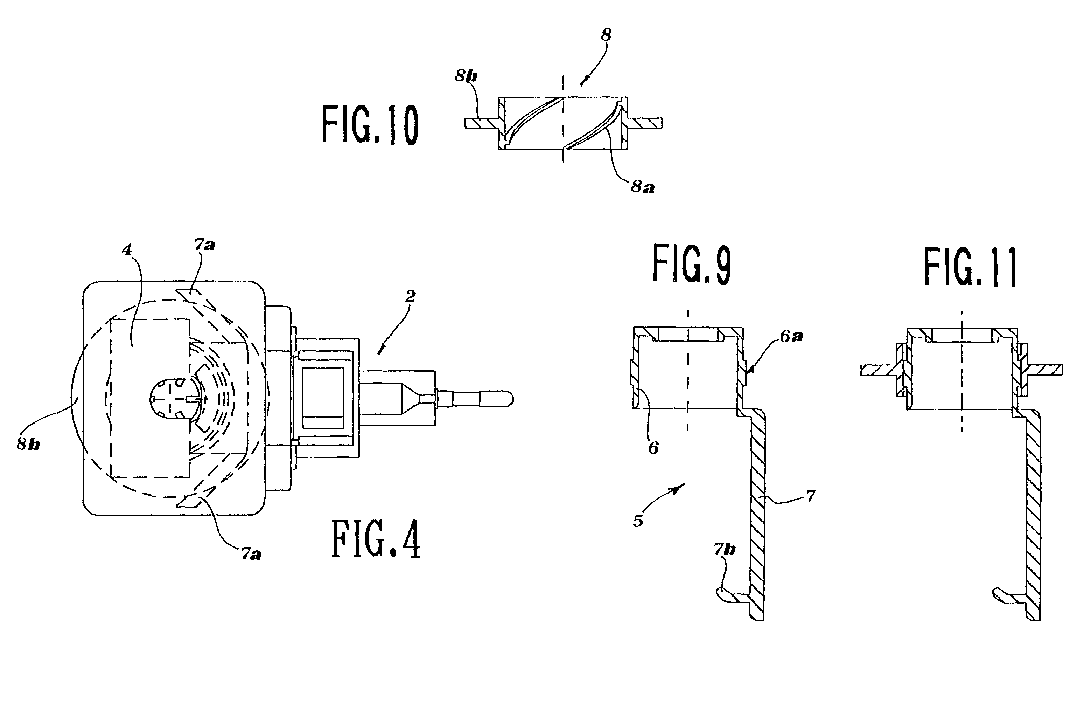

A heating device 4 is fixed by conventional means into the upper part of the housing 1, and preferably - but not necessarily - comprises a ceramic support incorporating a resistor or a posistor (PTC), electrically connected to the pins of the plug 2 through the switch 3. Electric connections of this type are widely known in technique and are thus not illustrated on the drawing to make it clearer. The shape of the heating device 4 - as shown in the plan view of fig. 4 - is such that the seat for the wick S of the bottle F has an open section, for example U-shaped, to allow an easy insertion of the bottle F, as better described hereinafter.

The central part of the housing 1 holds the unit supporting the bottle F, which consists of a bottle holder 5 and of a bush 8 controlling the same. The structure of these two elements of the evaporator is illustrated in further detail in figs. 5 to 11. As clearly shown on said drawings, the bottle holder 5 comprises a cylindrical portion 6 connected to a vertical side member 7, and preferably formed in one piece therewith through a conventional molding process of a plastic material. A helicoidal rib 6a is formed onto the outer surface of the cylindrical portion 6, in one piece therewith, while two wings 7a laterally project from the side member 7, with an inclination towards the front part of the evaporator, and a bracket 7b projects from the lower part of the evaporator. As shown in the cross section views - wherein, for simplicity, the wings 7a have been removed - the cylindrical portion 6 is pierced at the top, to allow the passage of the wick S of the bottle F, and is provided there with the usual means for centering the wick.

The control bush 8 consists of an annular cylindrical body, apt to engage with the cylindrical portion 6 and comprising, for the purpose, an inner helicoidal rib 8a, preferably formed in one piece with the bush 8. The pitch of the rib 8a obviously corresponds to that of the rib 6a, so as to ensure a proper engagement between the cylindrical portion 6 and the control bush 8. Said bush is moreover provided with an external flange 8b of wider diameter, kept in position into suitable seats formed therefor into the housing 1, and partially projecting from the front wall of said housing - as can be seen from figs. 1 and 4 - so that its rotation may be easily controlled by the user.

When the bottle holder 5 is inserted into the housing 1, the wings 7a projecting from the side member 7 get into sliding contact with the sidewalls of said housing, hence allowing the bottle holder 5 to perform only a rectilinear sliding motion in a direction parallel to the axis of the wick S. Any rotary motion of the control bush 8 thus turns - thanks to the screw-and-nut relationship between the ribs 8a and 6a - into a motion of axial translation of the entire bottle holder 5, thereby allowing to reach the object of the invention. As shown on the drawings, the rib 6a, unlike the rib 8a, has a remarkable extention in height; in this way, the rib 6a is in fact able to cooperate not only with the rib 8a but also with the whole inner wall of the control bush 8, thus providing a reliable, steady and smooth control of the movement of the bottle holder 5.

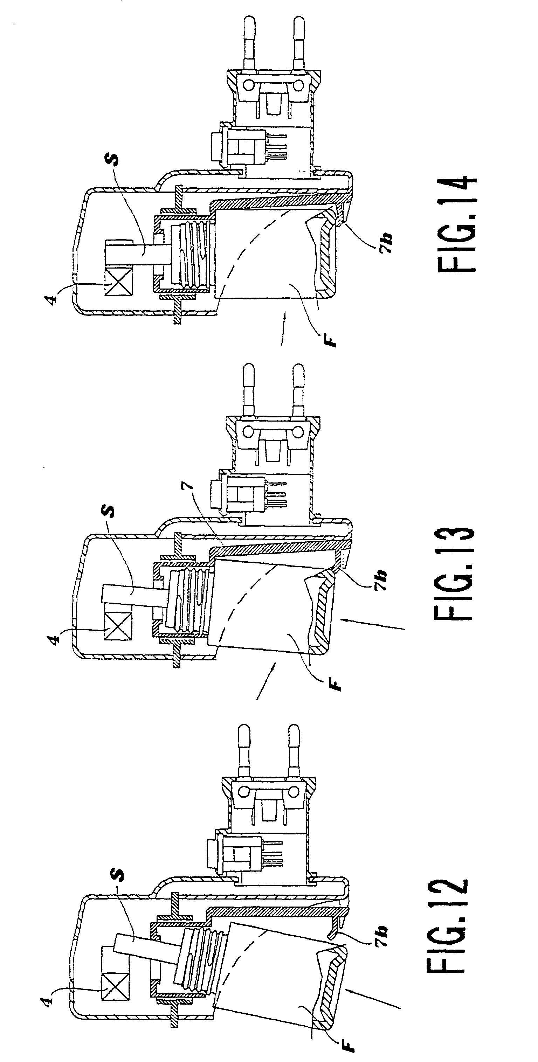

The mounting of the bottle F into the bottle holder 5 is shown in figs. 12 to 14. A first step (fig. 12) involves introducing the bottle F into the cylindrical portion 6 of the bottle holder 5, making sure to avoid the obstacle formed by the bracket 7b; in this step, the wick S, being inclined in respect of a vertical direction, can be easily inserted into the U-shaped seat provided therefor in the heating device 4. A second step (fig. 13) involves pushing the bottle F upwards, towards its vertical position, by pressing its lower portion against the bracket 7b and elastically deforming the side member 7 until the bottle has taken up a perfectly vertical position. In this last step (fig. 14), the bracket 7b - the positioning of which along the side member 7 is planned so as to exactly reproduce the size of the sidewall of the bottle F - is released from the edge of the bottle F and moves back to its initial position, thanks to the elasticity of the side member 7, fitting onto the bottom of the bottle F and thus positively blocking it inside the evaporator, independently from any subsequent handling, or even tilting, to which it will be subjected during use. To remove the empty bottle F, it will then be sufficient to manually shift the side member 7 in the position of fig. 14, grasp the bottle F and pull it downwards and in a direction away from the plug 2, so as to step back into the positions shown in fig. 13 and, respectively, fig. 12.

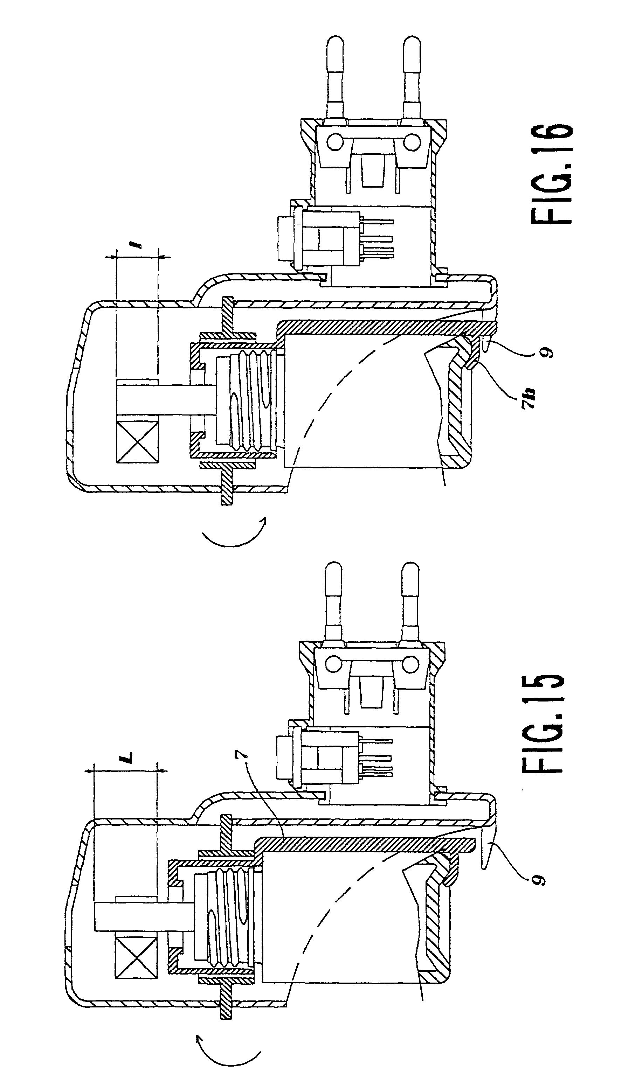

Figs. 15 and 16 finally show the two end positions of the bottle holder 5 - with the bottle F and the wick S inserted therein - determined by the rotation of the control bush 8. Fig. 15 shows the position of maximum evaporation, wherein the wick S is in its highest position and thus receives a higher amount of heat from the heating device 4. Since, in fact, the heat let out by said device produces a hot air stream flowing upwards, the wick S is affected by said stream throughout its length L. Acting now on the rotation of the control bush 8, the bottle holder 5 moves down, up to reaching the bottom end position shown in fig. 16, in which the stretch of wick influenced by the hot air stream is of reduced length I, and which thus corresponds to the position of minimum evaporation.

The upper end position of the bottle holder 5 is determined by the contact between the shoulder of the side member 7 and the bush 8, while the lower end position is determined by the contact between the bracket 7b and a pair of stop pins 9 projecting from the bottom part of the housing 1, the side member 7 being freely inserted between said stop pins, as shown in Fig. 2.

From the previous description, it appears quite evident how the present invention has fully reached the intended objects. The heretofore described evaporator allows in fact to use a bottle having any shape, even highly irregular, seen that said bottle is connected to the evaporator merely in correspondence of its neck (forcedly circular, as it is meant to house the closing cap) and of its bottom part. A bottle of this type can thus be freely moved in a rectilinear direction, parallel to the wick, so as to vary the stretch of wick affected by the hot air stream produced by the heating device 4 and thus, in the end, the amount of active substance spread out per unity of time.

Moreover, the configuration of the device is such that the bottle keeps in a steady position however the user may handle the evaporator. Equally steady is the axial position of the bottle, determined by the rotation of the control bush 8; in fact, since the screw-and-nut control of this latter is not of the reversible type, the position of the bottle F cannot be varied accidentally - for example, by imparting a pressure onto the bottom of the bottle - but only, and exclusively, through rotation of the annular flange 8b forming part of the control bush 8.

## Claims

1. Electric evaporator for insecticides or perfumes in liquid formulations - of the type comprising a housing (1), a rotatable plug (2), an electric heating device (4), and a bottle (F) containing the liquid formulation, provided with a wick (S), wherein said bottle (F) is held into a bottle holder (5), rectilinearly slidable in respect of the housing (1) in a direction parallel to the axis of the wick (S), **characterized in that** said bottle holder (5) also comprises a cylindrical portion (6) connected in a screw-and-nut relationship to a control bush (8), apt to be operated externally to the evaporator so as to control the rectilinear sliding motion of said bottle holder (5).

2. Electric evaporator as in claim 1), wherein said cylindrical portion (6) of the bottle holder (5) forms the seat for the bottle neck.

3. Electric evaporator as in claim 2), wherein said cylindrical portion (6) of the bottle holder (5) comprises an outer helicoidal rib (6a).

4. Electric evaporator as in claim 3), wherein said control bush (8) consists of an annular cylindrical body, externally engaging with the cylindrical portion (6) of the bottle holder (5), and comprises an inner helicoidal rib (8a) connected in a screw-and-nut relationship to the outer rib (6a) of said cylindrical portion (6).

5. Electric evaporator as in claim 4), wherein said control bush (8) comprises an external widened annular engagement flange (8b) projecting from the front wall of the housing (1) of the evaporator.

6. Electric evaporator as in claim 1), wherein the sliding portion of said bottle holder (5) consists of a side member (7), connected to said cylindrical portion (6), so as to be elastically deformable in respect thereof, in order to allow introducing and removing the bottle (F).

7. Electric evaporator as in claim 6), wherein said side member (7) comprises a projecting bracket (7b), apt to elastically engage the bottom of the bottle (F).

8. Electric evaporator as in claim 6), wherein said side member (7) is provided with two laterally projecting wings (7a), apt to slidingly engage with the inner sidewalls of the housing (1).

9. Electric evaporator as in claim 1), wherein said housing (1) comprises, in its bottom part, a pair of projecting pins (9), apt to form the lower stops for said bottle holder (5).

10. Electric evaporator as in claim 6), wherein said side member (7) comprises a shoulder which connects it to said cylindrical portion (6) and is apt to form the upper stop for said bottle holder (5) in abutment against said control bush (8).

## Patentansprüche

1. Elektrischer Verdampfer für Insektizide oder Parfüme in Flüssigformulierung von der Bauart mit einem Gehäuse (1), einem drehbaren Stecker (2), einer elektrischen Heizeinrichtung (4) und einer die Flüssigformulierung enthaltenden Flasche (F), die mit einem Docht (S) versehen ist, worin die Flasche (F) in einem Flaschenhalter (5) gehalten wird, der in Bezug auf das Gehäuse (1) in einer zur Achse des Dochtes (S) parallelen Richtung geradlinig gleitfähig ist, **dadurch gekennzeichnet, daß** der Flaschenhalter (5) auch einen zylindrischen Bereich (6) umfaßt, der in einer Schraube-und-Mutter-Beziehung mit einer Steuerbuchse (8) verbunden ist, die geeignet ist, um außerhalb des Verdampfers betätigt zu werden, um die geradlinige Gleitbewegung des Flaschenhalters (5) zu steuern.

2. Elektrischer Verdampfer nach Anspruch 1, **dadurch gekennzeichnet, daß** der zylindrische Bereich (6) des Flaschenhalters (5) den Sitz für den Flaschenhals bildet.

3. Elektrischer Verdampfer nach Anspruch 2, **dadurch gekennzeichnet, daß** der zylindrische Bereich (6) des Flaschenhalters (5) eine äußere spiralförmige Rippe (6a) umfaßt.

4. Elektrischer Verdampfer nach Anspruch 3, **dadurch gekennzeichnet, daß** die Steuerbuchse (8) aus einem ringförmigen zylindrischen Körper besteht, der außen mit dem zylindrischen Bereich (6) des Flaschenhalters (5) in Eingriff steht und eine innere spiralförmige Rippe (8a) umfaßt, die in einer Schraube-und-Mutter-Beziehung mit der äußeren Rippe (6a) des zylindrischen Bereiches (6) verbunden ist.

5. Elektrischer Verdampfer nach Anspruch 4, **dadurch gekennzeichnet, daß** die Steuerbuchse (8) einen äußeren aufgeweiteten ringförmigen Eingreifflansch (8b) umfaßt, der von der Vorderwand des Gehäuses (1) des Verdampfers vorragt.

6. Elektrischer Verdampfer nach Anspruch 1, **dadurch gekennzeichnet, daß** der Gleitbereich des Flaschenhalters (5) aus einem Seitenelement (7) besteht, das mit dem zylindrischen Bereich (6) verbunden ist, um in Bezug darauf elastisch verformbar zu sein und ein Einsetzen und Entfernen der Flasche (F) zu gestatten.

7. Elektrischer Verdampfer nach Anspruch 6, **dadurch gekennzeichnet, daß** das Seitenelement (7) einen vorragenden Halter (7b) umfaßt, der zum elastischen Eingreifen der Unterseite der Flasche (F) geeignet ist.

8. Elektrischer Verdampfer nach Anspruch 6, **dadurch gekennzeichnet, daß** das Seitenelement (7) mit zwei seitlich vorragenden Flügeln (7a) versehen ist, die zum gleitfähigen Eingriff mit den inneren Seitenwänden des Gehäuses (1) geeignet sind.

9. Elektrischer Verdampfer nach Anspruch 1, **dadurch gekennzeichnet, daß** das Gehäuse (1) in seinem unteren Teil ein Paar vorragende Stifte (9) umfaßt, die zur Bildung der unteren Anschläge für den Flaschenhalter (5) geeignet sind.

10. Elektrischer Verdampfer nach Anspruch 6, **dadurch gekennzeichnet, daß** das Seitenelement (7) eine Schulter umfaßt, die es mit dem zylindrischen Bereich (6) verbindet und geeignet ist, um den oberen Anschlag für den Flaschenhalter (5) bei Anliegen an der Steuerbuchse (8) zu bilden.

## Revendications

1. Evaporateur électrique pour insecticides ou parfums dans des formulations liquides - du type comportant un boîtier (1), une fiche tournante (2), un dispositif de chauffage électrique (4) et un flacon (F) contenant la formulation liquide, pourvu d'une mèche (S) dans lequel ledit flacon (F) est maintenu dans un support (5) de flacon, pouvant coulisser de façon rectiligne par rapport au boîtier (1) dans une direction parallèle à l'axe de la mèche (S), **caractérisé en ce que** ledit support (5) de flacon comporte aussi une partie cylindrique (6) reliée dans une relation de vis-et-écrou à une bague (8) de commande, pouvant être manoeuvrée extérieurement à l'évaporateur afin de commander le mouvement de coulissement rectiligne dudit support (5) de flacon.

2. Evaporateur électrique selon la revendication 1, dans lequel ladite partie cylindrique (6) du support (5) de flacon forme le siège pour le col du flacon.

3. Evaporateur électrique selon la revendication 2, dans lequel ladite partie cylindrique (6) du support (5) de flacon comporte une nervure hélicoïdale extérieure (6a).

4. Evaporateur électrique selon la revendication 3, dans lequel ladite bague (8) de commande consiste en un corps cylindrique annulaire, en prise extérieure avec la partie cylindrique (6) du support (5) de flacon, et comporte une nervure hélicoïdale intérieure (8a) reliée par une relation de vis-et-écrou à la nervure extérieure (6a) de ladite partie cylindrique (6).

5. Evaporateur électrique selon la revendication 4, dans lequel ladite bague de commande (8) comporte un rebord annulaire extérieur et élargi (8b) d'engagement faisant saillie de la paroi avant du boîtier (1) de l'évaporateur.

6. Evaporateur électrique selon la revendication 1, dans lequel la partie coulissante dudit support (5) de flacon consiste en un élément latéral (7), relié à ladite partie cylindrique (6), afin de pouvoir être déformé élastiquement par rapport à celle-ci, pour permettre l'introduction et l'enlèvement du flacon (F).

7. Evaporateur électrique selon la revendication 6, dans lequel ledit élément latéral (7) comporte une console (7b) en saillie, pouvant être engagée élastiquement contre le fond du flacon (F).

8. Evaporateur électrique selon la revendication 6, dans lequel ledit élément latéral (7) est pourvu de deux ailes (7a) faisant saillie latéralement, pouvant entrer en contact de glissement avec les parois latérales intérieures du boîtier (1).

9. Evaporateur électrique selon la revendication 1, dans lequel ledit boîtier (1) comporte, dans sa partie de fond, deux ergots (9) en saillie, pouvant former les butées inférieures dudit support (5) de flacon.

10. Evaporateur électrique selon la revendication 6, dans lequel ledit élément latéral (7) comporte un épaulement qui le relie à ladite partie cylindrique (6) et qui peut former la butée supérieure pour ledit support (5) de flacon en appui contre ladite bague (8) de commande.
